# EUROPEAN PATENT SPECIFICATION

(11) **EP 1 487 509 B1**
(45) Date of publication and mention of the grant of the patent: **11.11.2009**
(21) Application number: 03745065.7
(22) Date of filing: 26.03.2003
(51) Int. Cl.: A61L 15/60, A61F 13/534

(54) **ABSORBENT PRODUCT**
SAUGFÄHIGES PRODUKT
PRODUIT ABSORBANT

(30) Priority: 27.03.2002 SE 0200950
(43) Date of publication of application: 22.12.2004
(73) Proprietor: SCA Hygiene Products AB, 405 03 Göteborg (SE)
(72) Inventor: FALK, Torgny, S-422 41 Hisings-Backa (SE)
(74) Representative: Bergstrand, Mikael Gudmundsson
(86) International application number: PCT/SE2003/000501
(87) International publication number: WO 2003/080136

(56) References cited:
- EP-A1- 0 948 952
- EP-A2- 0 278 601
- WO-A1-90/14815
- WO-A1-91/11978
- WO-A1-98/06364
- US-A- 5 985 432

## Description

### Technical field

The invention refers to an absorbent product, such as a panty liner, a sanitary towel or an incontinence protection, for absorption of blood-containing fluids and urine, which product comprises an absorbent body comprising SAP-material.

### Background to the invention

Materials of superabsorbent polymers (SAP) have become popular for use in various absorbent products. SAP-material has the ability to absorb liquid in an amount several times its own weight, which makes SAP suitable for use as a component in for example an absorbent body in a panty liner, a sanitary towel or a diaper.

Typically, SAP is added to the remaining absorbent body in the form of polymer particles. These particles are built up of superabsorbent polymer chains forming a network, which is held together by cross-linkings. In order to obtain these cross-linkings cross-linking agents are used.

For example, DE-A-19941423 discloses a SAP-polymer and its manufacture, which comprises (a) 55-99.9 % (per weight) of an ethylene-containing polymerisable monomer, (b) 0-40 % (per weight) of a with (a) copolymerisable monomer, (c) 0.01-5.0, preferably 0.1-2.0 % (per weight), of a cross-linking agent, and (d) 0-30 % (per weight) of a water-soluble polymer.

The degree of cross-linking determines the elasticity of the material, as well as its absorbent properties. A higher degree of cross-linking results in a more brittle material, but also a higher initial ability to absorb fluid, i.e. the SAP becomes quick, but obtains a lower total capacity to absorb a large volume, i.e. a lower total capacity. A low degree of cross-linking gives a viscous, expandable material, which has the capacity to absorb a large volume of liquid. However, a low degree of cross-linking may result in a relatively slow absorption. A reason for this is that the diffusion constant increases and the swelling is faster (at a higher cross-linking degree), but the total capacity decreases ("Modern superabsorbent polymer technology", Buchholz and Graham, Wiley-VCH, 1998). A balance between these two extremes is therefore desirable.

A high degree of cross-linking also results in high gel strength. The gel strength is a measure of the ability of the SAP-gel to withstand pressure without losing fluid. A SAP-particle having a high gel strength thus has a high ability to keep fluid when it is subjected to load, such as when the wearer of an absorbent product sits down or lies in a way that the article, and the SAP-particle within it, is pressed together.

The fluids that must be absorbable by an absorbent product in the category of panty liners, sanitary towels, incontinence protections or tampons are primarily urine and blood-containing fluids, such as menstrual fluid. Urine is a water-based solution, which among others comprises various salts. Blood has a higher viscosity than urine. To about 45 % blood comprises of blood cells and to about 55 % of blood plasma. The blood plasma comprises inter alia salts, water and proteins (inter alia albumin, IgG and fibrinogen). The proteins constitute about 7 to 8 % of the blood plasma. The amount of proteins in blood is of the magnitude 10¹⁷/ml blood, and the amount of blood cells is in the magnitude of 10⁹ /ml blood.

At absorption of blood to a SAP-particle the proteins will interact to a much greater extent than the blood cells with the surface of the SAP-particles. The proteins give a negative effect on the absorption capacity of the SAP-particles. The SAP-particles are primarily intended for absorption of water. Thus it is important to design the surface of the SAP-particles in such a way that the transport of water molecules into the particles are facilitated. By making the surface of the SAP-particles hydrophilic, the ability of the proteins to interact with the surface of the particles will decrease. Tests (Nadarajah et al., "Modeling the Dynamics of Proteins Adsorption to Surfaces", from ACS Symposium Series 602, Proteins at Interfaces II - Fundamentals and Applications (Horbett and Brash, 1995): chapter 13) have shown that a hydrophilic surface results in a lower degree of protein interaction. However, the water molecules are polar and will thus interact to a high degree with a hydrophilic surface. The same counts for salts, which in water solution are present as ions, and also will be attracted by the surface of the SAP-particles. In this way the transport of water and salt to the surface of the SAP-particles, and thus their possibility to be absorbed into the SAP-particles, will increase at expense of the protein accumulation at the surface, if the surface of the SAP-particles is hydrophilic. Moreover, a large difference in ionic concentration inside and outside a SAP-particle may give a high osmotic pressure, which contributes to a high absorption capacity.

Accordingly, the various fluids to be absorbed by an absorbent product in the technical area of the invention are different in their chemical character.

Today, a number of products concerning absorption of fluids having different chemical character exist. WO97/13484 discloses a so-called pore gradient for absorption of bodily fluids. This pore gradient extends in the Z-direction, in the direction of thickness, and comprises several layers of absorbent material having varying pore sizes in order to absorb fluids in an effective way.

EP-A-1077051 discloses a panty liner built up by two material layers, wherein the lower layer comprises SAP-material. WO96/23474 teaches an absorbent product for absorption of bodily fluids. This product has essentially two components: a collection component and a storage component. The purpose of the collection component, which is positioned between the source of fluid (the carrier) and the storage component, is to collect for example blood cells, and to allow for the liquid phase to be transported to and stored in the storage component. The storage component comprises SAP. However, none of these documents discloses SAP-material, which is especially adapted for blood absorption.

WO90/14815 discloses an absorbent article comprising at least two mutually different superabsorbent particles which are positioned in two physically separated layers. However, the document does not disclose SAP materials that are especially adapted for both blood and urine absorption.

US-A-5985432 describes a porous absorbent material, which, due to its favourable wettability and low contact angle for blood containing fluids (0-40°), is adapted for blood absorption. Furthermore, US-A-5241009 describes an absorbent product, which may be used for absorption of blood. In this document a cross-linking degree of about 0.1-2.0 % is recommended for blood absorption. US-A-5985432 discloses a SAP having a cross-linking degree of 0.001 to 5 % (per mole). In this case, the absorbent material comprises a polyether and/or a polycation bound to the absorbent polymer, which results in a contact angle for blood in the interval from 0 to 40°. These SAP-materials, having a relatively low degree of cross-linking, are however primarily suitable for absorption of water or urine. Since these products are not constituted for facilitating absorption of blood, with the specific composition of blood, they tend to not be functionable in cases where both urine and blood must be absorbed.

EP-A-278601 discloses an absorbing composite material for use in an absorbent product. The composite material comprises a water-absorbing polymer, a water-insoluble inorganic material and a water-insoluble hydrophilic fibrous material. However, superabsorbent materials are not disclosed. One purpose of this disclosure is to absorb fluids of varying viscosity.

WO98/06364 discloses an absorbent macro-structure that comprises an aggregate of bound particles comprising cross-linked particles of e.g. an essentially water-insoluble, absorbent, hydrogel-forming polymer material. The purpose is to provide a material that avoids the problem of gel blocking, and optimises the permeability properties.

Accordingly, a number of technical solutions for absorption of blood and urine in the technical area exist. However, a problem not yet solved is to absorb blood and urine in the same absorbent body in an efficient way. Thus, there is a need for an absorbent body, which body is constituted in a way that both blood and urine may be absorbed in such an effective way that the risk for leakage is minimised.

The object of the present invention is to provide an absorbent product having an absorbent body, which body is constituted in such a way that both urine and blood-containing fluids is absorbed to a high extent.

### Summary of the invention

This and other objects are accomplished with the present invention, which discloses an absorbent product, such as a panty liner, a sanitary towel, an incontinence protection or a tampon for absorption of blood or menstrual fluid, which comprises a liquid-permeable surface layer, intended to face the user at use, a liquid-impermeable backing layer, intended to face away from the user at use, and an absorbent body, whereby the absorbent body comprises superabsorbent polymers (SAP), characterised by that the SAP-material is of at least two variants, wherein the first variant is a SAP having a cross-linking degree in the interval from 0.02 to 2.0 %, and the second variant is a SAP having a cross-linking degree in the interval from 3.0 to 7.0 %. Hereby, SAP-material adapted for absorption of urine is combined with SAP-material for absorption of blood-containing liquids.

In a preferred embodiment the absorbent body is constituted of an upper and a lower layer, whereby the upper layer comprises SAP-material which is adapted for blood absorption and the lower layer comprises SAP-material which is adapted for urine absorption. Hereby, a favourable absorption of both blood and urine is achieved, since the urine having a low viscosity easily is transported through the high cross-linking degree upper layer, to the lower layer having a high swell capacity.

### Short description of the drawings

Figure 1 shows a panty liner according to one embodiment of the invention, in which the absorbent body comprises two layers.
Figure 2 shows a cross-section of a fibre-structure, which after section is used as an absorbent body.
Figure 3 shows a cross-section of a principal outline of an absorbent body according to one embodiment of the invention, in which the absorbent body comprises two material layers.
Figure 4 shows an embodiment according to the invention having SAP-material in different zones.
Figure 5 shows another embodiment according to the invention having SAP-material in different zones.

### Detailed description of the invention

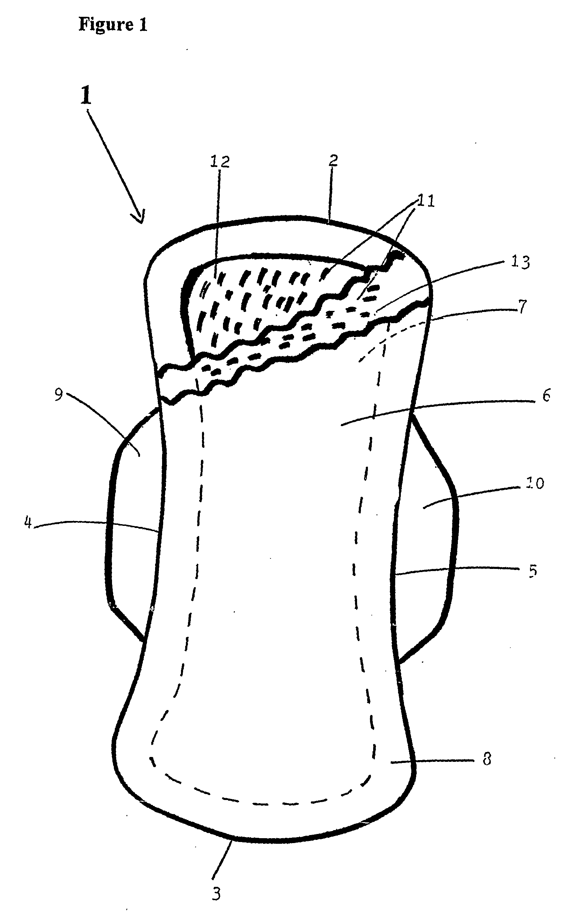

In figure 1 a principal sketch of a sanitary towel 1 according to the invention is shown. However, the invention is not limited to sanitary towels, but may also comprise other absorbent products, such as panty liners, incontinence protections and tampons, which are intended for absorption of blood, menstrual fluid and/or urine. In one embodiment the sanitary towel may include two short sides 2, 3 and two long sides 4, 5. A liquid permeable surface layer 6 is applied on the side of the sanitary towel 1 facing the wearer during use. The liquid permeable surface layer 6 suitable comprises of a soft, slcin-friendly material. Examples of suitable liquid permeable materials are different kinds of non-woven fibre materials. Other usable surface layers are perforated plastic films, nets, knitted, crocheted or woven textiles, and combinations and laminates of the listed material types.

The liquid-blocking backing layer 7 consists of a liquid-impermeable material. Thin, liquid-proof plastic films are suitable for the purpose, but it is also possible to use materials which initially are liquid-permeable, but which are equipped with a coating of plastics, resin, or some other fluid-proof material. Hereby, the leakage of fluid form the underside of the absorbent product is prevented. The blocking layer 7 may therefore comprise any material meeting the criteria of liquid-impermeability, and exhibiting an appropriate sufficient flexibility and skin-friendliness. Example of materials that are suitable for use as the blocking layer are plastic films, nonwovens and laminates of these. The plastic film may for example be of polyethylene, polypropylene, polyester or plastic fibres. The blocking layer may alternatively consist of a laminate of a liquid-permeable plastic layer, facing the absorbent body, and a non-woven facing the undergarment of the wearer. A construction of that kind provides a leakage-secure layer having a textile character. The fluid-blocking backing layer 7 may also consist of a vapour-permeable material. A breathable backing layer 7 of that kind may for example be a so-called SMS-material (spunbond-meltblown-spunbond) or a breathable plastic film consisting of polyethylene. A plastic film of that kind is disclosed in EP-A-283200. In order to uphold the breathability even when the material is applied on a product, the under-side should not be totally covered with fastening means.

The two wrapping layers (the surface layer and the backing layer 6, 7) may be connected to each other and optionally form a protruding connecting edge 8 around the contour line of the sanitary towel. The connection may be accomplished with any technique suitable for the purpose, such as gluing, welding or sewing. The two wrapping layers are not necessary, but may in some cases be suitable.

Between the surface layer and the backing layer 6, 7 a thin flexible absorbent core 11 is placed, which may comprise one or more material layers (12, 13). The absorbent core 11 is suitably manufactured by one or more layers of cellulose pulp. The pulp may originally be in the form of rolls, bales or sheets, which at the manufacture of the sanitary towel is dry-defibrated and transmitted in fluffed form to a pulp mat, sometimes including so-called superabsorbents, which are polymers having the ability to absorb water or bodily fluids in an amount of several times their own weight. An alternative to this is to dry-form a pulp mat, such as described in WO94/10956. Examples of other usable absorbent materials are different kinds of natural fibres, such as cotton fibres, peat or the like. Naturally, it is also possible to use absorbent synthetic fibres, or particles of a high-absorbing polymer material of the type, which at absorption chemically bind large amounts of liquid, during the formation of a liquid-containing gel, or mixtures of natural fibres or synthetic fibres. The absorbent body 11 may further comprise additional components, such as form-stabilising means, fluid-spreading means, or binders, such as for example thermoplastic fibres, which have been heat-treated to hold short fibres and particles to a connecting unit. It is also possible to use different types of absorbing foam materials in the absorbent body. Yet another variant is to produce the absorbent body of only SAP-material.

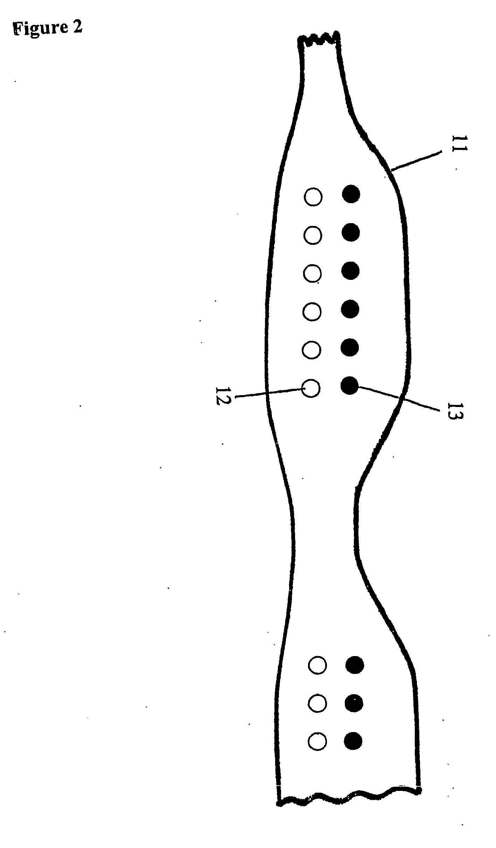

In figure 2 a cross-section of a fibre structure which is partially compressed is shown. The fibre structure may for example be of plastic, pulp, SAP or mixtures of these materials.

In a first embodiment of the invention the absorbent body comprises two different SAP-materials, whereby the first variant has a cross-linking degree in the interval from 0.02 to 2.0 %, and the other variant has a cross-linking degree in the interval from 3.0 to 7.0 %. Hereafter, the term "the first SAP-variant" refers to the SAP-variant having a low degree of cross-linking, and "the second SAP-variant" refers to the SAP-variant having a high degree of cross-linking. Thus, the first variant is a conventional SAP-material, having a high swell-capacity, and thereby suitable for absorption of urine. The second variant is the SAP-material disclosed in SE0103961-9 (filed November 27, 2001), which document hereby is incorporated as a reference. This second variant is especially suitable for the absorption of blood-containing fluids due to its high degree of cross-linking and its hydrophilic surface. In this way, an absorbent core is provided having the ability to absorb both blood and urine. Preferably, the first SAP-variant has a cross-linking degree in the interval from 0.1 to 1.0 %, and the second variant has a cross-linking degree in the interval from 4.0 to 5.0 %.

In the embodiment, the second SAP-variant, which is adapted for blood absorption, has a gel strength, which is higher than 18 kPa, preferably higher than 25 kPa, more preferably higher than 30 kPa. A SAP-material having a gel strength at this level have especially favourable blood absorption characteristics. For further details of the manufacture of SAP-particles of the second variant, as well as its absorption properties, see SE0103961-9. Typically, the first SAP-variant has a gel strength that is lower than 10 kPa, normally about 5 kPa.

The strength of the SAP-gel (shear modules) may for example be determined with a TA instruments AR 1000 N. For example, to 0.5 g SAP-sample 2.5 ml 0.9 % NaCl-solution is added. The elasticity module of the SAP-samples is determined with a TA instruments AR 1000 N. The analyses are performed with a 40 mm acrylic parallel plate, cross hatched at 20°C. The procedure is oscillating and the samples are analysed between 0,1 and 100 Hz. The oscillating shear tension is 10.00 Pa. The measured shear tension is designated G' and is measured at 1 Hz.

The superabsorbent polymers (SAP) of the invention are manufactured of polyacrylic acid monomers, which are provided from KeboLab, for instance. The monomers are thereafter treated in order to form polymers. They are added to the other absorption material in the form beads, granules, foam, fibres, threads, film or the like. Granules are preferred in the invention, and they are used at an approximate size of 100 to 850 µm. Naturally, other manufacturing methods may be used if they generate SAP:s having the above mentioned features.

Other materials that may be used to produce superabsorbent polymers suitable to use in the invention, include hydrolyzed starch-acrylonitrile graft copolymers, starch-acrylic acid graft copolymers, saponified vinyl acetate-acrylic ester copolymers, hydrolyzed acrylonitrile copolymers, hydrolyzed acrylamide copolymers, ethylene-maleic anhydride copolymers, isobutylene-maleic anhydride copolymers, poly(vinylsulfonic acid), poly(vinylphosphonic acid), poly(vinylphosphoric acid), poly(vinylsulfuric acid), sulfonated polystyrene, poly(aspartic acid), poly(lactic acid), and mixtures thereof, as well as mixtures of the above mentioned compounds and polyacrylic acid. Also other material combinations know in the technical area are of course possible to use, and are included in the scope of the invention.

The polymer chains are held together with cross-linkings, which are accomplished by the addition of a cross-linking agent, such as methyl-bisacrylamide (MBA), to the original polymers. Other possible cross-linking agents comprise for example aluminium sulphate, N,N'-methylenebisacrylamide, N,N'-methylenebismethacrylamide, ethylene glycol dimethacrylate, and trimethylolpropane triacrylate. It is to be understood by the skilled person that also other cross-linking agents giving a desired cross-linking are included in the scope of the invention.

According to the invention, the cross-linking agent is added to the SAP-polymer in a concentration of 0.02 to 2 % (per mole), preferably 0.1 to 1 % (per mole), for the first variant, and 3 to 7 % (per mole), preferably 4 to 5 % (per mole), for the second variant. For the second variant, these values have shown to result in especially advantageous absorption properties for blood or menstrual fluid. These values are measured at a load of 2 kPa, using the AUL-method (Absorption Under Load) in a AUL-cell, which has been modified by exchanging a standard filter to a metal filter (having a mesh size of 100 x 100 µm). These metal filters have enough large meshes for blood cells to pass (about 10 µm in diameter). The absorption capacity values mentioned here are measured according to edana 441.1-99 (centrifuge retention capacity).

The surface of the SAP-particles is treated in a way that it preferably exhibits a certain degree of wettability (hydrophilicity). The wettability is measured by the so-called contact angle. Defibrinogated sheep blood is standardly used to measure the contact angle for SAP-particles to be used for absorption of blood-containing fluids. The contact angle (see for example "Lexikon i KEMI", Gleerups förlag, 1976, 1^{st} edition) specifies the angle between the surface and a water droplet, positioned on the surface. The more hydrophilic surface, the more compressed the droplet becomes (smaller angle). A surface showing a value for the contact angle below 90° is regarded as hydrophilic. In an absorbent product according to the invention a contact angle for the second variant of SAP material, which is smaller than 60° and preferably smaller than 40°, is exhibited, as measured with defibrinogated sheep blood.

The contact angle between defibrinogated sheep blood and the SAP-particles may be determined with a DAT 1100 Fibro System AB. The analyses are for example performed in normal laboratory environment at 22 °C and 50 % relative air moisture. For the analyses a particle size of between 100 and 315 µm is for example chosen.

In order for the surface of the SAP-particles to exhibit the desired hydrophilicity, the surface is treated with a surface modifier, such as aluminium chloride (AlCl₃) and/or aluminium sulphate (Al₂(SO₄)₃). At treatment of a SAP-particle surface with these ionic compounds, the chloride- and/or sulphate ions will dissociate and a binding of the trivalent, positively charged aluminium ion is obtained on the surface. At absorption of water containing liquids, the water dipole will then be attracted to and bind to the aluminium ion. Other surface modifiers that may be used include silica compounds, such as silica oxides, organic compounds, such as ethylene carbonate, and tensides. All surface modifiers giving the desired effect, i.e. a desired contact angle, are fully possible for use in the invention.

A SAP-material according to the second variant may for example be treated and surface-modified as follows: To a 25 % acrylic acid-solution, neutralised to 75 %, methylenebisacrylamide is added to a cross-linking degree of 0.1 to 10 %. The reaction is initiated with 0.1 % mole of Va-044 (Wako Pure Chemical Industries LTD, Japan), and is neutralised with sodium ions (such as sodium hydroxide or sodium carbonate). The obtained gel is washed, dried, ground (Janke & Kunkel, Analysen Mühce A10) and fractionated to a size in the interval from 100 to 850 µm.

At surface-crosslinking the particles are evaporated in a solution of ethanol, aluminium chloride and sodium hydroxide. This is performed by mixing 25 ml 96 % ethanol, 0.1 g water-free AlCl₃ and 300 µl 4M NaOH with 2 g SAP. The mix is allowed to cross-link for about 30 min at 70°C, during rotation. Thereafter, the ethanol is removed under vacuum.

In a preferred embodiment according to the invention, the absorbent body essentially comprises an upper (13) and a lower layer (12), whereby each of the two SAP-variants are essentially present in one of the two layers. Hereby, one of the layers is especially adapted for absorption of blood and one is especially adapted for absorption of urine.

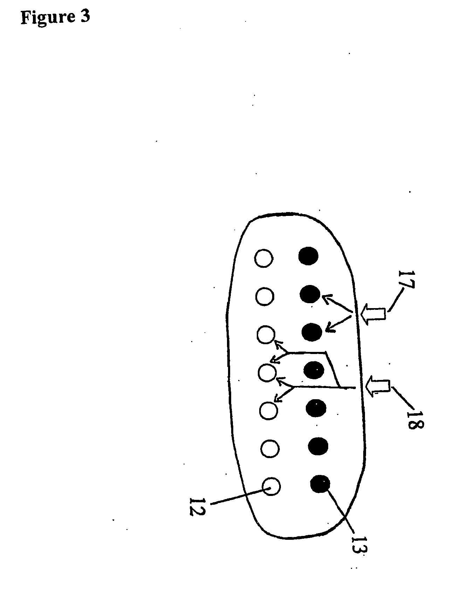

In an especially advantageous embodiment (figure 3) the SAP-material of the upper layer (13) is composed of the second SAP-variant, and the SAP-material of the lower layer (12) is composed of the first variant. Hereby, an absorbent body in the absorbent product is obtained, in which body the upper layer has a high degree of cross-linking and the lower layer has a low degree of cross-linking. Urine (18), which is a fluid of low viscosity will easily flow through the upper layer and primarily be absorbed by the lower layer. Blood-containing fluids (17), such as menstrual fluid, which fluids are relatively viscous, will be absorbed by the upper layer, and will thus essentially not flow down to the lower layer. Moreover, as the upper layer is strongly cross-linked the risk for gel blockage is reduced, since the strongly cross-linked polymers are not expanded in the same way as weakly cross-linked polymers, and will therefore not block the fluid transport.

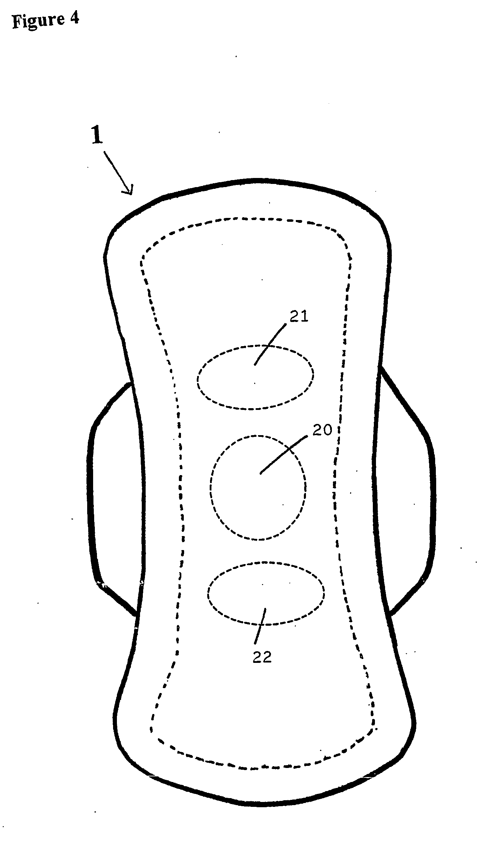

In the invention, both SAP-variants are arranged in such a way, that zones are formed in/at the absorbent body. For example, in one embodiment (figure 4) a zone of SAP-material of the second variant (high degree of cross-linking) is positioned in the centre of (longitudinally) the absorbent body (20), and one, two or more zones of SAP of the first variant (low degree of cross-linking) are positioned in front of (21) and/or behind (22) (longitudinally) the zone of the second variant (20). The purpose of this embodiment is to arrange the SAP-zones in such a way that they, at use, are close to the bodily parts of the wearer that gives off the bodily fluids that are to be absorbed, such as menstrual fluids and urine.

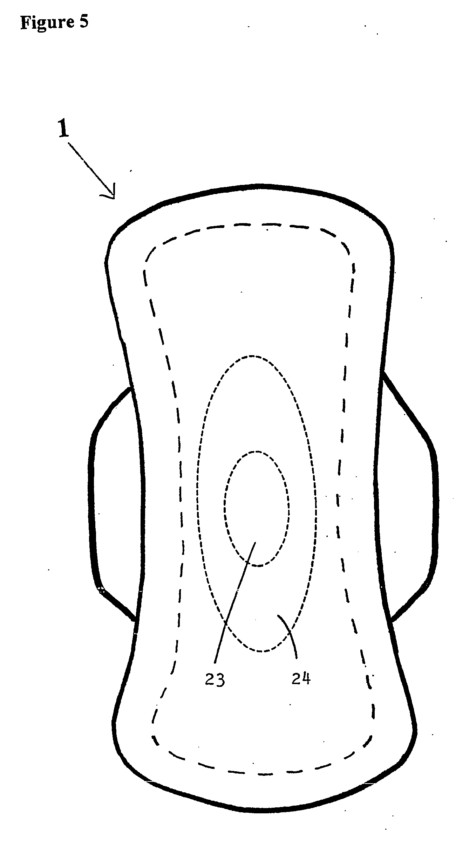

In another embodiment (figure 5), a zone of SAP of the second variant (23) is applied centrally on, at the middle of, the absorbent body, for example in the form of a circle, whereby SAP of the first variant is applied in a zone (24) around the SAP-zone of the second variant (23). The purpose is to arrange the SAP-variants in a way that for an absorption of the desired bodily fluids as effective as possible to be obtained.

In yet another embodiment both SAP-variants may be mixed in one layer, or in several layers, whereby for example the relative quantity ratio of the different variants of SAP-material is different in different layers, so that for example an upper layer comprises a majority of SAP of the second variant, and the lower layer comprises a majority of SAP of the first variant. Moreover, more than two layers may be used, whereby the various layers comprises different compositions of SAP-material.

Other variants and combinations of the above mentioned embodiments are also fully possible, as long as the absorbent body comprises SAP-material of at least two variants as described above.

A wearer suffering of for example mild incontinence will generally give off at least a few ml menstrual fluid and urine. According to a preferred embodiment the invention is thus designed sin such a way that the upper layer has a total absorption capacity of at least 2 ml, and the lower layer has a total absorption capacity of at least 2 ml. In this way 2 ml blood-containing fluid may preferably be absorbed by the upper layer, and 2 ml urine of the lower layer. In another embodiment the invention is designed in order for the both layers total absorption capacities taken together to be approximately 15-20 ml. Thus, the absorbent product of the invention is for example especially suitable for mildly incontinent persons also having menstruation, or for other wearers for whom both urine and blood are given off.

In one embodiment of the invention, the SAP-material of the second variant has an absorption capacity of at least 6 g blood/g SAP, more preferably of at least 10 g blood/g SAP, and most preferably of at least 13 g blood/g SAP under load, and the first variant has an absorption capacity of at least 20 g salt solution/g SAP, more preferably at least 30 g salt solution/g SAP, and most preferably at least 35 g salt solution/g SAP under load (AUL). By salt solution is here for example meant 0.9 % NaCl-solution, for example saline solution. The absorption capacity is measured according to edana 441.1-99 (centrifuge retention capacity).

The SAP-particles are added to the absorbent body in an amount of 1 to 100, preferably 10 to 60 % of the total weight of the absorbent body, in order to achieve a good spreading and/or absorption capacity.

Between the surface layer 6 and the absorbent body 11 an inlet layer may be arranged. The purpose of the inlet layer is to direct liquid into the absorbent product, and to transport it down to the absorbent body 11. The inlet layer may be a low-density non-woven material.

At the downside of the absorbent product a fastening means may be applied. The fastening means comprises preferably glue, but it may also be a mechanical fastening means such as hook and loop, push buttons, friction linings, clamping means, or the like. The glue may be applied in one or more strings, or in any other pattern. Alternatively, the whole downside of the absorbent product 1 is lined with glue. It is also possible to use a fastening glue, which is breathable, and to apply it to the entire downside of the product, for it to function as a combined liquid-proof layer and fastening means. Moreover, the fastening means may be glue towards body, hydrogels or nothing at all.

On the fastening means, a protection layer may be applied, e.g. when glue is used as the fastening means. The protection layer is preferably a siliconised paper, but also other variants of protection layers are of course possible, such as waxed papers, embossed or release agent-treated plastic film, textile ribbons to fasten to hooks and loops, etc.

The absorbent product of the invention may further also comprise fastening tabs 9 and 10, which are applied along the long sides of the absorbent product. The purpose of the tabs is for them to be folded along the edge of the panty, and thereby keep the absorbent product in place. Furthermore, other kinds of fastening systems may be used.

Primarily, the absorbent product of the invention is a panty liner, a sanitary towel, or an incontinence protection, adapted for absorption of urine, blood or menstrual fluid.

In yet another aspect, the invention relates to the use of an absorbent product described above for the absorption of both urine and blood-containing fluids.

## Claims

1. An absorbent product, such as a panty liner, a sanitary towel, an incontinence protection or a tampon for absorption of urine, blood or menstrual fluid, comprising a fluid permeable surface layer facing the wearer during use, a fluid impermeable backing layer facing away from the wearer during use, and an absorbent body, positioned between the surface layer and the backing layer, whereby the absorbent body comprises superabsorbent polymers (SAP), **characterised by** that the SAP-material is of at least two variants, whereby the first variant is a SAP with a cross-linking degree in the interval from 0.02 to 2.0 %, and the second variant is a SAP with a cross-linking degree in the interval from 3.0 to 7.0 %, whereby the second variant mainly is intended for blood absorption and has a gel strength higher than 18 kPa, and whereby the SAP-material is arranged in at least two zones in the absorbent body, whereby at least one zone essentially comprises SAP-material of the first variant, and at least one of the zones essentially comprises SAP-material of the second variant.

2. Absorbent product according to claim 1, whereby the first SAP-variant has a cross-linking degree in the interval from 0.1 to 1.0 %, and the second SAP-variant has a cross-linking degree in the interval from 4.0 to 5.0 %.

3. Absorbent product according to claim 2, whereby the second SAP-variant has a gel strength higher than 25 kPa.

4. Absorbent product according to claim 3, whereby the second SAP-variant has a gel strength higher than 30 kPa.

5. Absorbent product according to any one of the preceding claims, whereby the second SAP-variant shows a contact angle on its surface which is lower than 60°, preferably lower than 40°, as measured by defibrinogated sheep blood.

6. Absorbent product according to any one of the preceding claims, whereby the absorbent body essentially comprises an upper and a lower layer, whereby each of the two SAP-variants are essentially present in one of the two layers.

7. Absorbent product according to claim 6, whereby the SAP-material of the upper layer is composed of the second SAP-variant, and the SAP-material in the lower layer is composed of the first SAP-variant.

8. Absorbent product according to claim 6 or 7, whereby the upper layer has a total absorption capacity of at least 2 ml, and the lower layer has a total absorption capacity of at least 2 ml.

9. Absorbent product according to any one of claim 6-8, whereby the upper layer is adapted for absorption of blood-containing fluids, and the lower layer is adapted for absorption of urine.

10. Absorbent product according to any one of claim 1-9, whereby a zone of SAP-material of the second variant is positioned in the middle of the absorbent body, and that at least one zone of SAP-material of the first variant is positioned longitudinally in front of and/or behind, and/or around the zone of the second variant.

11. Absorbent product according to any one of the preceding claims, whereby the SAP-material of the second variant has an absorption capacity of at least 6 g blood/g SAP, preferably at least 10 g blood/g SAP, and more preferably at least 13 g blood/g SAP under load, and the first variant has an absorption capacity of at least 20 g salt solution/g SAP, preferably at least 30 g salt solution/g SAP, and more preferably at least 35 g salt solution/g SAP under load of 2 kPa.

12. Absorbent product according to any one of the preceding claims, whereby the amount of SAP-material of the absorbent body is in the interval from 1 to 100 %, preferably from 10 to 60 %.

## Patentansprüche

1. Absorptionsprodukt, wie zum Beispiel eine Slipeinlage, eine Binde, ein Inkontinenzschutz oder ein Tampon zur Absorption von Urin, Blut oder Menstruationsfluid, umfassend eine fluiddurchlässige Oberflächenschicht, die während der Verwendung dem Träger zugewandt ist, eine fluidundurchlässige Rückschicht, die während der Verwendung von dem Träger abgewandt ist, und einen Absorptionskörper, der zwischen der Oberflächenschicht und der Rückschicht positioniert ist, wobei der Absorptionskörper superabsorbierende Polymere (SAP) aufweist, **dadurch gekennzeichnet, dass** das SAP-Material aus zumindest zwei Varianten ist, wobei die erste Variante ein SAP mit einem Vernetzungsgrad im Intervall von 0,02 bis 2,0 % und die zweite Variante ein SAP mit einem Vernetzungsgrad im Intervall von 3,0 bis 7,0 % ist, wobei die zweite Variante hauptsächlich für Blutabsorption gedacht ist und eine Gelstärke von mehr als 18 kPa aufweist und wobei das SAP-Material in zumindest zwei Zonen in dem Absorptionskörper angeordnet ist, wobei zumindest eine Zone im Wesentlichen SAP-Material der ersten Variante umfasst und zumindest eine der Zonen im Wesentlichen SAP-Material der zweiten Variante umfasst.

2. Absorptionsprodukt nach Anspruch 1, wobei die erste SAP-Variante einen Vernetzungsgrad im Intervall von 0,1 bis 1,0 % aufweist und die zweite SAP-Variante einen Vernetzungsgrad im Intervall von 4,0 bis 5,0 % aufweist.

3. Absorptionsprodukt nach Anspruch 2, wobei die zweite SAP-Variante eine Gelstärke von mehr als 25 kPa aufweist.

4. Absorptionsprodukt nach Anspruch 3, wobei die zweite SAP-Variante eine Gelstärke von mehr als 30 kPa aufweist.

5. Absorptionsprodukt nach einem der vorgehenden Ansprüche, wobei die zweite SAP-Variante einen Kontaktwinkel, gemessen durch defibrinogiertes Schafblut, auf ihrer Oberfläche zeigt, der kleiner als 60°, bevorzugt kleiner als 40° ist.

6. Absorptionsprodukt nach einem der vorgehenden Ansprüche, wobei der Absorptionskörper im Wesentlichen eine obere und eine untere Schicht aufweist, wobei jede der zwei SAP-Varianten im Wesentlichen in einer der zwei Schichten vorliegt.

7. Absorptionsprodukt nach Anspruch 6, wobei das SAP-Material der oberen Schicht aus der zweiten SAP-Variante aufgebaut ist und das SAP-Material in der unteren Schicht aus der ersten SAP-Variante aufgebaut ist.

8. Absorptionsprodukt nach Anspruch 6 oder 7, wobei die obere Schicht eine Gesamtabsorptionskapazität von mindestens 2 ml hat und die untere Schicht eine Gesamtabsorptionskapazität von mindestens 2 ml hat.

9. Absorptionsprodukt nach einem der Ansprüche 6 bis 8, wobei die obere Schicht zur Absorption von Blut enthaltenden Fluiden angepasst ist und die untere Schicht zur Absorption von Urin angepasst ist.

10. Absorptionsprodukt nach einem der Ansprüche 1 bis 9, wobei eine Zone aus SAP-Material der zweiten Variante in der Mitte des Absorptionskörpers positioniert ist und die zumindest eine Zone von SAP-Material der ersten Variante in Längsrichtung vor und/oder hinter und/oder um die Zone der zweiten Variante herum positioniert ist.

11. Absorptionsprodukt nach einem der vorgehenden Ansprüche, wobei das SAP-Material der zweiten Variante eine Absorptionskapazität von mindestens 6 g Blut/g SAP, bevorzugt mindestens 10 g Blut/g SAP und weiter bevorzugt mindestens 13 g Blut /g SAP unter Last aufweist und die erste Variante eine Absorptionskapazität von mindestens 20 g Salzlösung/g SAP, bevorzugt mindestens 30 g Salzlösung/g SAP und weiter bevorzugt mindestens 35 g Salzlösung/g SAP unter Last von 2 kPa aufweist.

12. Absorptionsprodukt nach einem der vorgehenden Ansprüche, wobei die Menge von SAP-Material des Absorptionskörpers im Intervall von 1 bis 100 %, bevorzugt von 10 bis 60 % ist.

## Revendications

1. Article absorbant tel qu'un protège slip, une serviette hygiénique, une protection contre l'incontinence ou un tampon, destiné à l'absorption d'urine, de sang ou de fluide menstruel, comportant une couche superficielle perméable aux fluides orientée vers l'utilisateur lors de l'utilisation, une couche arrière imperméable aux fluides orientée à l'écart de l'utilisateur lors de l'utilisation, et un corps absorbant positionné entre la couche superficielle et la couche arrière, le corps absorbant comprenant des polymères superabsorbants (SAP), **caractérisé par le fait que** le matériau SAP relève d'au moins deux variantes, la première variante étant un SAP doté d'un degré de réticulation compris dans l'intervalle allant de 0,02 % à 2,0 % et la deuxième variante étant un SAP doté d'un degré de réticulation compris dans l'intervalle allant de 3,0 % à 7,0 %, la deuxième variante étant principalement destinée à l'absorption du sang et présentant une force de gel supérieure à 18 kPa, et le matériau SAP étant disposé en au moins deux zones dans le corps absorbant, au moins une des zones comprenant essentiellement du matériau SAP de la première variante et au moins une des zones comprenant essentiellement du matériau SAP de la deuxième variante.

2. Article absorbant selon la revendication 1, dans lequel la première variante de SAP a un degré de réticulation compris dans l'intervalle allant de 0,1 % à 1,0 %, et la deuxième variante de SAP a un degré de réticulation compris dans l'intervalle allant de 4,0 % à 5,0 %.

3. Article absorbant selon la revendication 2, dans lequel la deuxième variante de SAP a une force de gel supérieure à 25 kPa.

4. Article absorbant selon la revendication 3, dans lequel la deuxième variante de SAP a une force de gel supérieure à 30 kPa.

5. Article absorbant selon l'une quelconque des revendications qui précèdent, dans lequel la deuxième variante de SAP présente un angle de contact sur sa surface qui est inférieur à 60°, de préférence inférieur à 40°, mesuré au moyen de sang de mouton défibriné.

6. Article absorbant selon l'une quelconque des revendications qui précèdent, dans lequel le corps absorbant comprend essentiellement une couche supérieure et une couche inférieure, chacune des deux variantes de SAP étant essentiellement présente dans l'une des deux couches.

7. Article absorbant selon la revendication 6, dans lequel le matériau SAP de la couche supérieure est constitué de la deuxième variante de SAP, et le matériau SAP de la couche inférieure est constitué de la première variante de SAP.

8. Article absorbant selon la revendication 6 ou 7, dans lequel la couche supérieure a une capacité d'absorption totale d'au moins 2 ml, et la couche inférieure a une capacité d'absorption totale d'au moins 2 ml.

9. Article absorbant selon l'une quelconque des revendications 6 à 8, dans lequel la couche supérieure est apte à absorber des fluides contenant du sang, et la couche inférieure est apte à absorber de l'urine.

10. Article absorbant selon l'une quelconque des revendications 1 à 9, dans lequel une zone de matériau SAP de la deuxième variante se situe au milieu du corps absorbant et au moins une zone de matériau SAP de la première variante se situe longitudinalement en face de et/ou derrière et/ou autour de la zone de la deuxième variante,

11. Article absorbant selon l'une quelconque des revendications qui précèdent, dans lequel le matériau SAP de la deuxième variante a une capacité d'absorption d'au moins 6 g de sang/g de SAP, de préférence d'au moins 10 g de sang/g de SAP, et plus préférablement d'au moins 13g de sang/g de SAP sous charge, et la première variante a une capacité d'absorption d'au moins 20 g de solution saline/g de SAP, de préférence d'au moins 30 g de solution saline/g de SAP, et plus préférablement d'au moins 35 g de solution saline/g de SAP sous une charge de 2 kPa.

12. Article absorbant selon l'une quelconque des revendications qui précèdent, dans lequel la quantité de matériau SAP du corps absorbant se situe dans l'intervalle compris entre 1 % et 100 %, de préférence entre 10 % et 60 %.
